# FASCICULE DE BREVET EUROPEEN

(11) **EP 1 251 841 B1**
(45) Date de publication et mention de la délivrance du brevet: **15.06.2005**
(21) Numéro de dépôt: 01902215.1
(22) Date de dépôt: 31.01.2001
(51) Int. Cl.: A61K 31/125, A61P 37/02

(54) **SOLUTION AQUEUSE DE CAMPHRE POUR LE TRAITEMENT DE MALADIES DEGENERATIVES OU AUTOIMMUNES ET COMME AGENT IMMUNOMODULATEUR**
CAMPHER WÄSSRIGE LÖSUNG ZUR BEHANDLUNG DEGENERATIVER UND AUTOIMMUNER ERKRANKUNGEN SOWIE ALS IMMUNOMODULATOR
CAMPHOR AQUEOUS SOLUTION FOR TREATING DEGENERATIVE OR AUTOIMMUNE DISEASES AND AS IMMUNOMODULATORY AGENT

(30) Priorité: 03.02.2000 CA 2297998
(43) Date de publication de la demande: 30.10.2002
(73) Titulaire: Naessens, Gaston, Rock Forest, Québec J1N 3B6 (CA)
(72) Inventeur: Naessens, Gaston, Rock Forest, Québec J1N 3B6 (CA)
(74) Mandataire: Bernasconi, Jean Raymond
(86) Numéro de dépôt international: PCT/CA2001/000108
(87) Numéro de publication internationale: WO 2001/056559

(56) Documents cités:
- WO-A-97/05780
- GB-A- 385 148
- F. MAHLA ET AL.: "Ueber einige Umwandlungsproducte des Campheroxims und Fenchonoxims" BERICHTE DER DEUTSCHEN CHEMISCHEN GESELLSCHAFT, vol. 29, no. 3, 1896, pages 2807-2808, XP002167940 Weinheim (DE)

## Description

La présente invention a pour objet une solution aqueuse qui est injectable par voie périnodulaire ou inhalable et est destinée à être utilisée pour le traitement de maladies dégénératives ou autoimmunes ou comme agent immunomodulateur.

L'invention a également pour objet un procédé de préparation de cette solution.

Ce procédé est caractérisé en ce que:
dans une première étape, on fait réagir du camphre avec de l'hydroxyde d'ammonium;
dans une seconde étape, on met le produit obtenu dans la première étape en suspension dans une solution aqueuse de chlorure de sodium; et
dans une troisième étape, on neutralise le pH de la suspension obtenue à la deuxième étape avec de l'acide nitrique pour obtenir la solution aqueuse désirée.

La solution aqueuse selon l'invention telle qu'elle est obtenue par le procédé ci-dessus décrit, a été abondamment testée et s'avère présenter des propriétés pharmacologiques la rendant efficace pour le traitement de maladies dégénératives ou autoimmunes ou comme agent immunomodulateur.

Ainsi, il a été noté qu'elle mime les cytokines humaines. Elle agit alors sur les monocytes pour les transformer en macrophages, lesquels secrètent alors deux cytokines proinflammatoires : l'interleukine 1 bêta, 6, 8α et un facteur de nécrose tumorale TNF alpha. Dépendant de la famille des cytokines mimées, les monocytes sont transformés en macrophages ou bloquent les molécules qui paralysent le système immunitaire. Dans les deux cas, il se produit une stimulation du système immunitaire et une augmentation du facteur de nécrose tumorale qui est aussi classé parmi les immunostimulants. Ce facteur est aussi connu pour le rôle qu'il joue dans la résistance de l'hôte aux infections virales ou autres et dans le développement des tumeurs.

Sur la base d'essais effectués sur des animaux, la solution selon l'invention serait applicable en thérapeutique humaine à raison de 0.075 ml par libre de poids corporel pour 21 jours, soit un total de 10,5 ml par série, ce qui correspond à 0.5 ml par jour pour une personne pesant 140 libres. L'administration peut s'effectuer par voie périnodulaire ou par inhalation au moyen d'un nébuliseur ultrasonique.

Pour une personne de 140 à 190 livres, la première série doit être appliquée de manière progressive selon le calendrier suivant :
1^{er} jour injection de 0.1 ml
2^{e} jour injection de 0.2 ml
3^{e} jour injection de 0.3 ml
4^{e} jour injection de 0.4 ml
5^{e} jour injection de 0.5 ml

Toutes les autres injections doivent comporter le même volume (0.5 ml) de produit injecté.

Les séries subséquentes utiliseront 0.5 ml à chaque injection pendant 21 jours.

Les cycles pourront être répétés au besoin avec un arrêt de 2 jours entre chaque cycle.

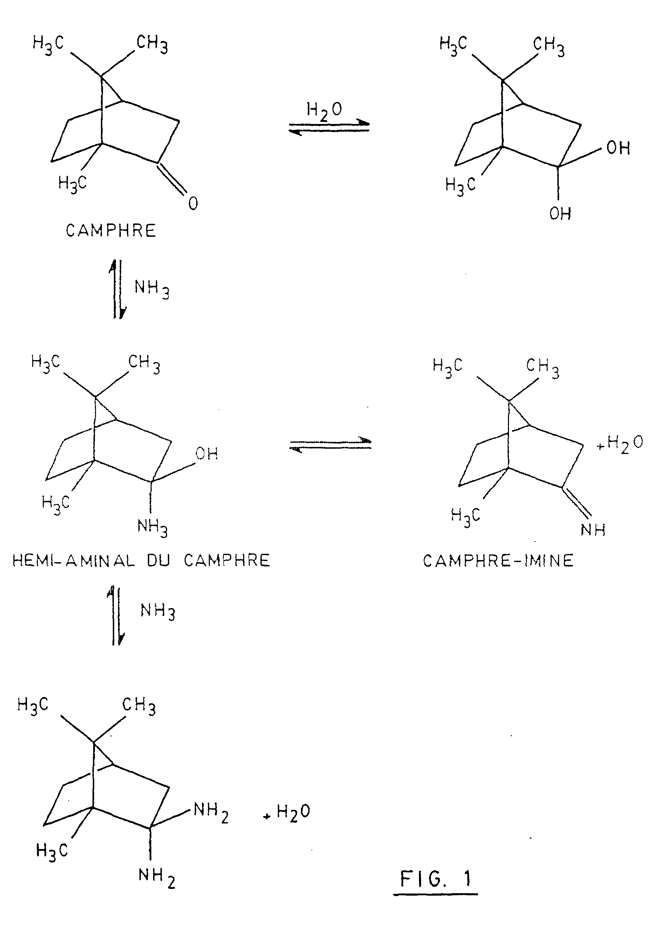

La structure du principe actif présent au sein de la solution aqueuse selon l'invention n'a pas encore été établie avec précision. Tel qu'il ressort sur le schéma réactionnel identifié comme Figure 1 et joint en annexe, le camphre réagit de façon réversible avec l'ammoniaque pour produire un dérivé hémi-aminal. Ce dérivé hémi-aminal du camphre est lui-même sujet à plusieurs conversions réversibles possibles en présence d'ammoniaque et d'eau, et a été jusqu'à présent impossible à identifier par spectrophotométrie infra-rouge. Le Demandeur pense toutefois que c'est le chlorure de ce dérivé hémi-aminal du camphre obtenu lors de la seconde étape de neutralisation qui est probablement le principe actif de la solution aqueuse selon l'invention dans la mesure où la structure de ce dérivé pourrait effectivement mimer la structure de cytokines de la famille β qui sont connues pour agir sur les monocytes et stimuler la production de IL 1 béta, 6, 8 et TNF alpha. Le nom chimique complet de ce chlorure de ce dérivé est le chlorure de triméthyl-1,7,7 amino-2 hydroxy-2 bicyclo [1,2,2] heptane.

L'invention sera mieux comprise à la lecture de ce qui va suivre d'un exemple pratique de synthèse et de la description détaillée des essais effectués jusqu'à ce jour par le Demandeur.

### EXEMPLE

Dans un récipient fermant hermétiquement, on additionne 108 mg de camphre (C₁₀ H₁₆O) à 0,9 ml d'alcool éthylique (C₂H₅O), jusqu'à dissolution complète. On additionne ensuite la solution alcoolique ainsi obtenue à 5,2 ml d'hydroxyde d'ammonium (NH₄OH). Le mélange obtenu est agité.

Dans un autre erlenmeyer, on dissout 0,9 g de chlorure de sodium (NaCl) dans 79 ml d'eau stérile et apyrogène. Le contenu de cet autre erlenmeyer est additionné au mélange précédemment préparé. Le nouveau mélange obtenu est agité fortement.

La solution aqueuse ainsi préparée présente un précipité floconneux et surnageant. Après trois jours à la température de la pièce et agitation quotidienne, le précipité est entièrement dissous.

Cette solution se présente sous la forme d'un liquide incolore, d'odeur ammoniacale et de saveur alcaline, dont le pH est de 10,4. On ajuste alors le pH à 7 en introduisant 14,9 ml d'acide nitrique (HNO₃) /N₆.

La solution finale obtenue est alors filtrée sur filtre millipore de 0,20 micron.

Il est bien entendu que les produits chimiques de base utilisés et précédemment mentionnés répondent aux normes U.S.P. et sont manipulés dans des conditions d'asepsie totale.

### PROPRIÉTÉS BIOLOGIQUES

Les propriétés biologiques qui ont été obtenues avec la solution préparée telle que décrite dans l'exemple qui précède, sont les suivantes:
1) Elle agit sur les monocytes in vitro.
2) Elle transforme les monocytes en macrophages matures in vitro
3) In vitro également, les macrophages transformés sont stimulés pour secréter des cytokines proinflammatoires.
   (a) Interleukine-1 bêta (IL-1 bêta) connue pour produire une grande variété d'effets sur la différentiation et la fonction des cellules impliquées dans les processus inflammatoires et les réponses immunitaires; et
   (b) IL-6,8 alpha et facteur de nécrose tumorale (TNF alpha) aussi classé comme immunostimulant et connu pour jouer un rôle dans la résistance de l'hôte aux infections et au développement des tumeurs.

Il est connu que la réponse immunitaire des cellules est contrôlée et modulée par une famille de relativement petites molécules appelées « cytokines » qui sont de petites hormones protéiques qui jouent un rôle dans de nombreuses fonctions cellulaires normales. Leurs fonctions englobent l'activité anti-tumorale, anti-virale, anti-bactérienne et provoquent la croissance des cellules immunitaires, la différentiation, l'activation, le chimiotactisme, l'adhésion et l'immunosuppression.

Cette famille d'immunomodulateurs a été découverte récemment. Plus de 70 molécules différentes ont été identifiées mais il semble que la famille comprend plus de 200 membres. En d'autres mots, les cytokines ne sont, à ce jour, que partiellement connues et caractérisées.

Celles connues ont été divisées en deux groupes, alpha et bêta, basés sur leur structure. Les cytokines alpha comprennent les Interleukines, interférons, et autres facteurs de croissance qui contrôlent la prolifération des cellules immunitaires. Les bêta cytokines comprennent les MIP (Macrophage Inflammatory Protein), les MCP (Monocyte Chemoattractant Protein), les RANTES, et autres protéines qui attirent les cellules immunitaires vers un foyer d'infection ou une tumeur et de cette manière renforcer l'activité du système immunitaire. Il semble que chaque molécule de cytokine soit très spécifique et cible seulement une petite sous-population de lymphocytes. De plus, des fragments de cytokines, aussi petits que 3-7 amino acides peuvent s'attacher aux lymphocytes et mimer partiellement ou bloquer l'activité d'une molécule complète de cytokine.

Il est aussi connu que les trois derniers amino acides du C-terminal des bêta cytokines sont nitrogène-oxygène qui ont une charge positive. Il est donc possible que la solution aqueuse selon l'invention ait un effet stimulateur pour secréter des cytokines du fait que, de par sa préparation, elle puisse contenir une petite quantité de molécules nitrogène-oxygène qui ressemblent et miment la séquence de 3 amino acides de la famille des bêta cytokines. Dépendant de l'activité des cytokines requise, la solution selon l'invention peut activer la transformation des monocytes en macrophages actifs ou bloquer l'action des autres molécules qui paralysent le système immunitaire. Le résultat dans chaque cas serait un renforcement du système immunitaire et des défenses naturelles pour augmenter la destruction des cellules tumorales.

### RÉSULTATS DES ESSAIS EFFECTUÉS

Divers essais et épreuves rapportés ci-après ont été effectués avec des ampoules remplies de la solution aqueuse préparée selon l'exemple ci-dessus donné.

### 1) Essais de contrôle de la solution :

La solution aqueuse préparée selon l'invention a été vérifiée. En pratique, elle doit être limpide, incolore et volatile et laisser un extrait sec de 63 mg par ml.

### 2) Essais de stérilité :

Dix ampoules choisies au hasard ont été placées à 37 degrés pendant 48 heures. Puis le contenu de chaque ampoule a été ensemencé comme suit :
a) 1 ml de solution respectivement dans deux tubes de 60 ml de milieu thioglycollate (Difco) et un tube de 60 ml de milieu liquide Sabouraud (Difco).
b) 0,25 ml de solution dans respectivement deux tubes de 60 ml de milieu thioglycollate (Difco) et un tube de 60 ml de milieu liquide Sabouraud (Difco).
c) Un tube de thioglycollate du groupe A et un tube de thioglycollate du groupe B furent placés dans une étuve à 35 degrés pendant 10 jours, tous les autres tubes étant gardés pendant 10 jours à la température de la pièce (approximativement 20 degrés).

Tous les tubes ensemencés sont demeurés stériles.

### c) Essais de toxicité :

Les essais pharmacologiques effectués sur des animaux sains ont montré une absence de toxicité, même à doses très élevées.

Ainsi, par voie intraveineuse, on a pu injecter 1 ml de produit à un lapin de 9 livres sans que l'on puisse observer une réaction défavorable. On a pu de la même façon injecter 0,2 ml de produit par livre de poids corporel à des chats et 0,3 ml de produit par litre de poids corporel à des chiens sans observer de réactions défavorables.

Par voie lymphatique, on a pu injecter à des chats de 10 à 12 livres jusqu'à deux fois la dose totale prévue en thérapeutique humaine, pour un poids corporel de 140 à 190 livres, sans observer de réactions défavorables.

### 4) Essais thérapeutiques :

L'activité thérapeutique du produit a été observée pendant près de trois ans sur 26 chats et 20 chiens atteints de diverses pathologies dégénératives ainsi que d'infections virales ou bactériennes.

Les doses unitaires injectées ont varié de un vingtième jusqu'au total de la dose ci-dessus proposée en thérapeutique humaine.

L'activité thérapeutique du produit a pu être observée dans tous les cas dès le deuxième jour du traitement. Cette activité s'est manifestée soit par une régression des masses tumorales ou ganglionnaires, soit par une reprise des fonctions vitales et un retour à un état général satisfaisant pour les maladies infectieuses.

Les résultats optimums ont été enregistrés avec une posologie de 0, 075 ml par livre de poids corporel, pour un cycle de 21 jours, ce qui a permis d'établir la posologie précédemment donnée applicable en thérapeutique humaine.

### 5) Essais pharmacologiques

Tel que précédemment indiqué, la solution aqueuse selon l'invention agit sur les cellules néoplasiques et empêche ces dernières de sécréter une substance qui rend les leucocytes et autres éléments phagocytaires de l'organisme en état de chimiotactisme négatif.

La suppression de cette sécrétion permet alors au système immunitaire de considérer la formation néoplasique comme un corps étranger et de la détruire. L'expérimentation animale a montré que l'élimination des masses tumorales lysées s'effectue par les émonctoires. Les cellules cancéreuses ainsi évacuées présentent une désorganisation nucléaire rendant toute mitose impossible. Ces cellules .évacuées sont entourées d'une quantité énorme et variée de leucocytes très actifs.

L'expérimentation animale a aussi montré que les constantes hématologiques des animaux traités se normalisent dès la première semaine de traitement.

La solution aqueuse selon l'invention peut être utilisée telle quelle, de préférence par voie lymphatique.

Du point de vue pratique, il est à signaler que cette solution ne doit pas être exposée aux rayons de tube germicides (rayon de 2537 A par exemple).

## Revendications

1. Méthode de préparation d'une solution aqueuse qui est injectable par voie périnodulaire ou inhalable et est utilisable pour le traitement de maladies dégénératives ou autoimmunes ou comme agent immunomodulateur, **caractérisée en ce que**:
- dans une première étape, on fait réagir du camphre avec de l'hydroxyde d'ammonium;
- dans une seconde étape, on met le produit obtenu dans la première étape en suspension dans une solution aqueuse de chlorure de sodium; et
dans une troisième étape, on neutralise le pH de la suspension obtenue à la deuxième étape avec de l'acide nitrique pour obtenir la solution aqueuse désirée.

2. Méthode selon la revendication 1, **caractérisée en ce que** dans la première étape, l'on fait réagir le camphre avec l'hydroxyde d'ammonium en milieu alcoolique.

3. Méthode selon la revendication 2, **caractérisée en ce que**:
- dans la première étape, on fait réagir 108 mg de camphre dans 0,9 ml d'alcool éthylique avec 5,2 ml d'hydroxyde d'ammonium;
- dans la seconde étape, on dissout la totalité du produit obtenu dans la première étape dans une solution contenant 79 ml d'eau et 0,9 g de chlorure de sodium; et
- dans la troisième étape, on ajuste le pH 7 par addition de 14,9 ml d'acide nitrique ayant une concentration N/6.

4. Méthode selon la revendication 1, 2 ou 3, **caractérisée en ce que** la solution aqueuse obtenue à la troisième étape est filtrée sur un filtre millipore.

5. Solution aqueuse injectable par voie périnodulaire ou inhalable pour le traitement de maladies dégénératives ou autoimmunes ou comme agent immunomodulateur, telle que préparée selon l'une quelconque des revendications 1 à 4.

6. Usage de la solution aqueuse selon la revendication 5 pour la fabrication d'un médicament pour le traitement de maladies dégénératives ou autoimmunes.

7. Usage de la solution aqueuse selon la revendication 5 comme agent immunomodulateur dans la fabrication d'un médicament.

## Patentansprüche

1. Verfahren zur Herstellung einer wäßrigen Lösung, die auf perinodalem Weg injizierbar oder inhalierbar ist und zur Behandlung degenerativer oder Autoimmunerkrankungen oder als Immunmodulator verwendbar ist, **dadurch gekennzeichnet, daß**:
- in einem ersten Schritt Campher mit Ammoniumhydroxid umgesetzt wird;
- in einem zweiten Schritt das im ersten Schritt erhaltende Produkt in wäßriger Natriumchloridlösung suspendiert wird und
- in einem dritten Schritt der pH der im zweiten Schritt erhaltenen Suspension mit Salpetersäure unter Erhalten der gewünschten wäßrigen Lösung neutral gestellt wird.

2. Verfahren gemäß Anspruch 1, **dadurch gekennzeichnet, daß** im ersten Schritt der Campher mit dem Ammoniumhydroxid in alkoholischem Medium umgesetzt wird.

3. Verfahren gemäß Anspruch 2, **dadurch gekennzeichnet, daß**:
- im ersten Schritt 108 mg Campher in 0,9 ml Ethylalkohol mit 5,2 ml Ammoniumhydroxid umgesetzt werden;
- im zweiten Schritt das gesamte, im ersten Schritt erhaltene Produkt in einer 79 ml Wasser und 0,9 g Natriumchlorid enthaltenden Lösung gelöst wird und
- im dritten Schritt der pH durch Zugabe von 14,9 ml Salpetersäure mit der Konzentration N/6 auf 7 eingestellt wird.

4. Verfahren gemäß einem der Ansprüche 1, 2 oder 3, **dadurch gekennzeichnet, daß** die im dritten Schritt erhaltene wäßrige Lösung durch ein Milliporefilter filtriert wird.

5. Auf perinodalem Weg injizierbare oder inhalierbare wäßrige Lösung zur Behandlung degenerativer oder Autoimmunerkrankungen oder als Immunmodulator, hergestellt gemäß einem der Ansprüche 1 bis 4.

6. Verwendung der wäßrigen Lösung gemäß Anspruch 5 zur Herstellung eines Arzneimittels zur Behandlung degenerativer oder Autoimmunerkrankungen.

7. Verwendung der wäßrigen Lösung gemäß Anspruch 5 als Immunmodulator bei der Herstellung eines Arzneimittels.

## Claims

1. Method for preparing an aqueous solution, which is injectable by perinodular injection or inhalable and is usable for the treatment of degenerative or autoimmune diseases or as an immunomodulatory agent, **characterised in that**
• in a first step, camphor is caused to react with ammonium hydroxide;
• in a second step, the product obtained in the first step is put into suspension in an aqueous solution of sodium chloride; and
• in a third step, the pH of the suspension obtained in the second step is neutralised with nitric acid to obtain the desired aqueous solution.

2. Method according to Claim 1, **characterised in that** in the first step, the camphor is caused to react with the ammonium hydroxide in an alcohol medium.

3. Method according to Claim 2, **characterised in that**:
• in the first step, 108 mg of camphor is caused to react with 5.2 ml of ammonium hydroxide in 0.9 ml of ethyl alcohol;
• in the second step, the whole of the product obtained in the first step is dissolved in a solution containing 79 ml of water and 0.9 g of sodium chloride; and
• in the third step, the pH 7 is adjusted by adding 14.9 ml of nitric acid with an N/6 concentration.

4. Method according to Claim 1, 2 or 3, **characterised in that** the aqueous solution obtained in the third step is filtered through a Millipore filter.

5. Aqueous solution that is injectable by perinodular injection or inhalable for the treatment of degenerative or autoimmune diseases or as an immunomodulatory agent, as prepared according to any one of Claims I to 4.

6. Use of the aqueous solution according to Claim 5 for the production of a medication for the treatment of degenerative or autoimmune diseases.

7. Use of the aqueous solution according to Claim 5 as immunomodulatory agent in the production of a medication.
